(19) Europäisches Patentamt / European Patent Office / Office européen des brevets

(11) **EP 4 130 286 A1**

(12) **EUROPEAN PATENT APPLICATION**
published in accordance with Art. 153(4) EPC

(43) Date of publication:
**08.02.2023 Bulletin 2023/06**

(21) Application number: **21781755.0**

(22) Date of filing: **02.04.2021**

(51) International Patent Classification (IPC):
**C12P 21/00** (1980.01)     **A23L 5/00** (2016.01)
**A61K 38/50** (1995.01)

(52) Cooperative Patent Classification (CPC):
**A23L 5/00; A61K 38/50; C12P 21/00**

(86) International application number:
**PCT/JP2021/014322**

(87) International publication number:
**WO 2021/201277 (07.10.2021 Gazette 2021/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.04.2020 JP 2020067751**

(71) Applicants:
• **Amano Enzyme Inc.**
**Nagoya-shi**
**Aichi 460-8630 (JP)**

• **Amano Enzyme U.S.A. Co., Ltd.**
**Elgin, Illinois 60124 (US)**

(72) Inventors:
• **OKUDA, Keita**
**Elgin, Illinois 60124 (US)**
• **SAKAI, Kiyota**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **PROTEIN DEAMIDATION METHOD**

(57)     The present invention addresses the problem of promoting a deamidation reaction with a protein deamidation enzyme. The present invention aims to promote the deamidation reaction by reacting a protein deamidation enzyme with a protein in the presence of a salt or a polysaccharide. According to the present invention, the reaction efficiency can be improved. The present invention is especially useful for the deamidation of a vegetable protein such as a pea protein. A deamidated protein (especially a deamidated vegetable protein) produced by employing the present invention can be used in various foods or beverages.

EP 4 130 286 A1

**Description**

TECHNICAL FIELD

The present invention relates to deamidation of a protein. Specifically, the present invention relates to a protein deamidation method and uses thereof, an agent for modifying protein used in a protein deamidation method, and the like.

BACKGROUND ART

Against the background of the recent increase in health consciousness, demand for vegetable proteins and the like has increased significantly. On the other hand, deamidation of a protein by a protein-deamidating enzyme improves solubility of the protein and imparts functionality as an emulsifier and a foaming agent, and thus is expected to be used in various applications including foods and beverages (see, for example, Patent Document 1).

PRIOR ART DOCUMENT

PATENT DOCUMENT

Patent Document 1: Japanese Patent Laid-open Publication No. 2000-50887

SUMMARY OF THE INVENTION

PROBLEMS TO BE SOLVED BY THE INVENTION

Since the deamidation reaction using the protein-deamidating enzyme is an enzyme reaction, the reaction may be insufficient due to the influence of conditions such as a substrate and a reaction temperature. In other words, since the reaction is an enzyme reaction, the desired effect (improvement of the functionality of the protein by deamidation, etc.) may not be obtained. Therefore, an object of the present invention is to provide a method for promoting a deamidation reaction by a protein-deamidating enzyme.

MEANS FOR SOLVING THE PROBLEM

As a result of intensive studies to solve the above problems, the present inventors have found that a crosslinking reaction of a protein is promoted when a protein-deamidating enzyme is allowed to act in the presence of a salt and/or a polysaccharide and have completed the present invention shown below.

[1] A protein deamidation method comprising allowing a protein-deamidating enzyme to act on a protein in the presence of a salt and/or a polysaccharide.
[2] The protein deamidation method according to [1], wherein the salt is at least one selected from the group consisting of carbonates, phosphates, hydrogen phosphates, polyphosphates, and citrates.
[3] The protein deamidation method according to [1] or [2], wherein the polysaccharide is gellan gum.
[4] The protein deamidation method according to any one of [1] to [3], wherein the protein-deamidating enzyme is protein glutaminase.
[5] The protein deamidation method according to any one of [1] to [4], wherein the protein is a vegetable protein and/or an animal protein.
[6] An agent for modifying protein comprising a salt and/or a polysaccharide and a protein-deamidating enzyme.
[7] The agent for modifying protein according to [6], wherein the salt is a phosphate, a polyphosphate, or a citrate.
[8] The agent for modifying protein according to [6] or [7], wherein the polysaccharide is gellan gum.
[9] The agent for modifying protein according to any one of [6] to [8], wherein the protein-deamidating enzyme is protein glutaminase.
[10] A method for producing a deamidated protein, including the steps of:

    (1) preparing a solution containing a protein and containing a salt and/or a polysaccharide; and
    (2) treating the prepared solution with a protein-deamidating enzyme.

[11] A method for producing a food or a medicine, including the steps of:

    (1) preparing a solution containing a food material or a pharmaceutical material and containing a salt and/or a polysaccharide; wherein the food material or pharmaceutical material contains a protein and
(2) treating the prepared solution with a protein-deamidating enzyme.

[12] The production method according to [10] or [11], wherein the salt is at least one selected from the group consisting of carbonates, phosphates, hydrogen phosphates, polyphosphates, and citrates.
[13] The production method according to any one of [10] to [12], wherein the polysaccharide is gellan gum.
[14] The production method according to any one of [10] to [13], wherein the protein-deamidating enzyme is protein glutaminase.
[15] The production method according to any one of [10] to [14], wherein the protein is a vegetable protein and/or an animal protein.

BRIEF DESCRIPTION OF THE DRAWINGS

Fig. 1 shows the results of the measurement of the deamidation reaction efficiency obtained by performing a reaction between benzyloxycarbonyl-L-glutaminylglycine (Z-Gln-Gly) and protein glutaminase using a solvent obtained by supplementarily adding sodium dihydrogen phosphate or sodium carbonate to a solution containing disodium hydrogen phosphate.
Fig. 2 shows the results of the measurement of the deamidation reaction effect obtained by performing a reaction between various substrate proteins and protein glutaminase using a solvent obtained by supplementarily adding sodium carbonate to a solution containing disodium hydrogen phosphate.
Fig. 3 shows the results of the measurement of the thermal stability of protein glutaminase in a solvent obtained by supplementarily adding sodium carbonate to a solution containing disodium hydrogen phosphate.

EMBODIMENT OF THE INVENTION

1. Protein Deamidation Method

A first aspect of the present invention relates to a method for deamidating a protein (protein deamidation method). In the present invention, the protein deamidation reaction is promoted by treating a protein material with a protein-deamidating enzyme in the presence of a salt and/or a polysaccharide.
The protein (substrate protein) to be subjected to the enzyme treatment in the present invention is not particularly limited as long as it is subjected to the action of the enzyme, and there is no particular restriction on its origin, property, and the like. The protein may be any of a vegetable protein, an animal protein, a protein derived from a fungus or a bacterium, a protein derived from algae, and the like.
Examples of the vegetable protein include proteins derived from beans such as soybeans, green peas, lentils, chickpeas, black beans, and fava beans; proteins derived from cereals such as wheat, barley, oat, rye, and rice; proteins derived from nuts such as almond and peanut; and proteins derived from seeds such as hemp seeds, chia seeds, quinoa, and amaranthus.
The animal protein may be derived from either a mammal or an insect. Examples of the protein derived from a mammal include milk proteins such as casein and $\beta$-lactoglobulin; egg proteins such as ovalbumin; meat proteins such as myosin and actin; blood proteins such as serum albumin; and tendon proteins such as gelatin and collagen. Examples of the protein derived from an insect include proteins derived from crickets.
Examples of the protein derived from a fungus or a bacterium include proteins derived from yeasts, proteins derived from filamentous fungi, and proteins derived from fungi (mycoproteins derived from mushrooms, etc.).
Among these substrate proteins, vegetable proteins and animal proteins are preferable as an object of deamidation, and vegetable proteins are more preferable.
In addition, the substrate protein may be a protein chemically partially decomposed by an acid, an alkali, or the like, a protein enzymatically partially decomposed by a protease or the like, a chemically modified protein by various reagents, a synthetic peptide, or the like.
The substrate protein may be used singly, or two or more substrate proteins may be used in combination.
The substrate protein as described above is subjected to a reaction in the form of a solution, slurry, or paste. The concentration thereof is not particularly limited, and the concentration may be determined according to the desired property and state of the target deamidated protein. In addition, the reaction is not limited to an aqueous solution, and a solution, a slurry, or a paste that is in an emulsion with oil or fat may be subjected to the reaction. Furthermore, a salt, a saccharide, a protein, a flavor, a moisturizer, a colorant, and the like may be added to the solution, slurry, or paste of the substrate protein as necessary.

The salt used in the present invention is preferably a water-soluble salt. The kind of the water-soluble salt is not particularly limited, and examples thereof include inorganic salts such as sulfate, hydrochloride, nitrate, dioxide, phosphate, hydrogen phosphate, polyphosphate, thiocyanate, thiosulfate, carbonate, and hydrogen carbonate and organic acid salts such as acetate, tartrate, citrate, and sorbate. The kind of metal atoms constituting the water-soluble salt is not particularly limited, and examples thereof include alkali metals such as sodium and potassium; alkaline earth metals such as magnesium and calcium; and other metals such as manganese, copper, and zinc. Among them, an alkali metal is preferable.

Among the salts, polyphosphate may be linear, branched, or cyclic but is preferably cyclic. The degree of polymerization of phosphoric acid in polyphosphate is not particularly limited and is, for example, 2 to 1,000, preferably 3 to 100, more preferably 4 to 10, still more preferably 5 to 7. Among polyphosphates, hexametaphosphate is preferable.

Preferable examples of the salt used in the present invention include phosphate, hydrogen phosphate, polyphosphate, citrate, sorbate, and carbonate, and more preferable examples include potassium phosphate, dipotassium hydrogen phosphate, disodium hydrogen phosphate, sodium dihydrogen phosphate, trisodium phosphate, sodium polyphosphate, trisodium citrate, and sodium carbonate. In particular, when carbonate is used as the salt, the deamination of the substrate protein can be performed remarkably efficiently, and further, the thermal stability of the protein-deamidating enzyme can be improved.

These salts may be used singly, or two or more salts may be used in combination. One example of a combination of two salts is a combination of hydrogen phosphate and carbonate. When hydrogen phosphate and carbonate are used in combination, the ratio thereof is not particularly limited, but for example, the amount of carbonate is 4 to 46 parts by weight, preferably 15 to 43 parts by weight, more preferably 20 to 41 parts by weight, per 1 part by weight of hydrogen phosphate.

The polysaccharide used in the present invention is preferably a water-soluble polysaccharide. The kind of the water-soluble polysaccharide is not particularly limited, and examples thereof include starch, dextrin, dextran, cellulose, xylan, tamarind seed gum, guar gum, tara gum, locust bean gum, gum arabic, carrageenan, gellan gum, xanthan gum, karaya gum, pectin, polygalacturonic acid, and alginic acid. These polysaccharides may be in the form of salts. Examples of the salt of the polysaccharides include alkali metal salts such as a sodium salt and a potassium salt.

Among these polysaccharides, acidic polysaccharides (polysaccharides having a carboxy group in at least one of constituent monosaccharides) are preferable, carrageenan, gellan gum, xanthan gum, karaya gum, pectin, polygalacturonic acid, and alginic acid are more preferable, and gellan gum is still more preferable.

The protein-deamidating enzyme referred to in the present invention is an enzyme that deamidates an amide group of a glutamine residue and an asparagine residue in a protein, and examples thereof include the followings.

(1) An enzyme that deamidates a glutamine residue in a protein and converts it into glutamic acid (such as protein glutaminase).
(2) An enzyme that deamidates an asparagine residue in a protein and converts it into aspartic acid (such as protein asparaginase).
(3) An enzyme that deimidates an arginine residue in a protein and converts it into citrulline (such as arginine deiminase, protein arginine deiminase, and peptidylarginine deiminase).

In general, when glutamine and asparagine residues in a protein are deamidated to generate carboxy groups, the negative charge of the protein increases, resulting in a decrease in the isoelectric point and an increase in the hydration force. Furthermore, an increase in electrostatic repulsive force leads to a decrease in interaction between protein molecules, that is, a decrease in associative properties. These changes greatly increase the solubility and water dispersibility of the protein. In addition, the increase in the negative charge of the protein loosens the folded protein, changes the higher-order structure, and exposes the hydrophobic region buried inside the molecule to the molecular surface. Therefore, the deamidated protein has amphiphilicity and becomes an ideal surfactant, and the emulsifying power, emulsion stability, foaming property, and foam stability of the protein are greatly improved. As described above, deamidation of a protein leads to improvement of various functional properties of the protein, and the use of the protein is dramatically increased. Also when an arginine residue in a protein is deiminated, the hydrophobicity of the protein is increased to change the higher-order structure of the protein.

Examples of the protein-deamidating enzyme include protein-deamidating enzymes derived from the genus Chryseobacterium, the genus Flavobacterium, the genus Empedobacter, the genus Sphingobacterium, the genus Aureobacterium, and the genus Myroides. These protein-deamidating enzymes are disclosed in Japanese Patent Laid-open Publication No. 2000-50887, Japanese Patent Laid-open Publication No. 2001-218590, WO 2006/075772, and the like.

Specific examples of the enzyme that deamidates the side chain of a glutamine residue in a protein include protein glutaminase derived from the genus Chryseobacterium.

Specific examples of the enzyme that deamidates an asparagine residue in a protein include protein-deamidating enzymes derived from the genus Luteimicrobium, the genus Agromyces, the genus Microbacterium, and the genus Leifsonia. These protein-deamidating enzymes are disclosed, for example, in WO 2015/133590.

Specific examples of the enzyme that deiminates arginine residues in a protein include arginine deiminase derived from the genus Fusarium. This protein-deamidating enzyme is disclosed, for example, in WO 2008/000714.

Examples of these protein-deamidating enzymes include preferably protein glutaminase, more preferably protein glutaminase derived from the genus Chryseobacterium, still more preferably protein glutaminase derived from Chryseobacterium proteolyticum. Protein glutaminase derived from Chryseobacterium proteolyticum is commercially available, for example, as Protein Glutaminase "Amano" 500 (manufactured by Amano Enzyme Inc.), and this commercially available product can be used.

In addition, the protein-deamidating enzyme may be an enzyme modified by a protein engineering technique.

As the protein-deamidating enzyme, one prepared from a culture solution of a microorganism that produces the protein-deamidating enzyme can be used. The microorganism to be used for the preparation of the protein-deamidating enzyme is not particularly limited but is a microorganism that produces the enzyme, and for example, a microorganism belonging to the genus Chryseobacterium, Flavobacterium, Empedobacter, Sphingobacterium, Aureobacterium, or Myroides can be used. In addition, a microorganism into which the gene encoding the protein-deamidating enzyme has been introduced by genetic engineering may be used. Specific examples of the microorganism suitable for preparation of the protein-deamidating enzyme include Chryseobacterium sp. No. 9670 belonging to the genus Chryseobacterium.

For example, the protein-deamidating enzyme can be obtained from the culture solution or bacterial cell of the microorganism. That is, a secretory protein can be recovered from the culture solution, and the other proteins can be recovered from the bacterial cells. As a method for preparing the protein-deamidating enzyme from the culture solution, a known protein separation and purification method (centrifugation, UF concentration, salting-out, various types of chromatography using an ion exchange resin, etc.) can be used. For example, a target enzyme can be obtained by centrifuging the culture solution to remove bacterial cells and performing a combination of salting-out, chromatography, and the like. In the case of recovering the enzyme from the bacterial cells, for example, the bacterial cells are crushed by pressurization treatment, ultrasonic treatment, or the like and then separated and purified in the same manner as described above, whereby the target enzyme can be obtained. The above-mentioned series of steps (crushing, separation, and purification of bacterial cells) may be carried out after the bacterial cells are previously recovered from the culture solution by filtration, centrifugation, or the like. The enzyme may be powdered by a drying method such as freeze drying or reduced pressure drying, or an appropriate excipient or drying aid may be used at that time.

As the protein-deamidating enzyme used in the present invention, it is desirable to use a purified protein-deamidating enzyme having a high purity, but the protein-deamidating enzyme may have any purity as long as it catalyzes a desired reaction. In addition, these enzyme preparations may be those to which various salt, saccharide, protein, lipid, and surfactant are added as enzyme stabilizer.

The enzyme activity of protein glutaminase, which is one of the protein-deamidating enzymes, is measured by the method described below using Z-Gln-Gly (Benzyloxycarbonyl-L-glutaminylglycine) as a substrate unless otherwise specified.

<Activity Measurement Method>

To 100 $\mu$l of a 176-mmol/l phosphate buffer solution (pH 6.5) containing 10 mmol/l of Z-Gln-Gly, 10 $\mu$l of an enzyme solution is added, and the mixture is incubated at 37°C for 60 minutes. Then, 100 $\mu$l of a 12% trichloroacetic acid solution is added to stop the reaction. After centrifugation (15,000 rpm, 4°C, 5 min), the supernatant is measured as follows using Ammonia Assay Kit (Sigma-Aldrich) (A1). Separate measurement is performed in the same manner using water instead of the enzyme solution (A2). To 100 $\mu$l of F-kit Ammonia Reagent 2, 10 $\mu$l of the supernatant and 190 $\mu$l of water are added, the mixture is allowed to stand at room temperature for 5 minutes, and then the absorbance (E1) at 340 nm is measured using 100 $\mu$l of the mixture. After 1.0 $\mu$l of Reagent 3 (glutamate dehydrogenase) is added to the remaining 200 $\mu$l, the mixture is further allowed to stand at room temperature for 20 minutes, and then the absorbance (E2) of the remaining 200 $\mu$l at 340 nm is measured. The amount of the enzyme releasing 1 $\mu$mol of ammonia per minute under the above conditions is defined as one unit and is determined according to the following formula.

$$U/ml = 1.76 \times [A1\ (E1 - E2) - A2\ (E1 - E2)]$$

Deamidation of a protein is performed by incubating a reaction solution containing the protein, a salt and/or a polysaccharide, and a protein-deamidating enzyme.

As long as the effect of the present invention, that is, the deamidation reaction of the protein, is promoted, the conditions of the treatment with the protein-deamidating enzyme are not particularly limited, and the protein concentration, the reaction temperature, the reaction pH, the reaction time, the addition amount of the salt (in the case where a salt is added), the addition amount of the polysaccharide (in the case where a polysaccharide is added), the addition amount of the enzyme (enzyme concentration), and the like may be adjusted through preliminary experiments to set optimal reaction conditions according to the enzyme to be used.

The concentration of the protein falls within, but is not limited to, 0.1 to 30 wt%, preferably 0.5 to 20% wt%, more preferably 0.9 to 11 wt% in the reaction solution. The amount of the deamidating enzyme is 0.01 to 1,000 U, preferably 0.1 to 100 U, more preferably 0.5 to 20 U, still more preferably 1 to 10 U, per 1 g of the protein. The concentration of the salt is 0.01 to 20% (W/V%), preferably 0.05 to 15% (W/V%), more preferably 0.1 to 11% (W/V%), in the reaction solution. The concentration of the polysaccharide is 0.001 to 10% (W/V%), preferably 0.005 to 5% (W/V%), more preferably 0.01 to 1% (W/V%), in the reaction solution. The reaction temperature is 5 to 80°C, preferably 20 to 70°C, more preferably 30 to 60°C. The pH of the reaction solution is 2 to 10, preferably 4 to 8. The reaction time is 10 seconds to 48 hours, preferably 10 minutes to 24 hours, more preferably 30 minutes to 12 hours, still more preferably 30 minutes to 4 hours. A deamidated protein can be obtained under the above reaction conditions. The conditions of these reactions are appropriately selected according to the physical properties of the protein and the salt, the polysaccharide, and the protein-deamidating enzyme to be used. The optimum reaction conditions may be determined through a preliminary experiment.

2. Method for Producing Deamidated Protein or Food and Pharmaceutical Product Containing Deamidated Protein

When the protein deamidation method of the present invention is used, a deamidated protein, or a food or a pharmaceutical product containing the deamidated protein can be produced. Therefore, the present invention also provides a method for producing a deamidated protein and a method for producing a food or a pharmaceutical product containing a deamidated protein. A typical aspect of the method for producing a deamidated protein includes the following steps (1) and (2). A deactivation step of the protein-deamidating enzyme may be added after the step (2). Specific conditions of the following steps (1) and (2) are as described in the section "1.1. Protein Deamidation Method" above.

(1) Preparing a solution containing a protein and containing a salt and/or a polysaccharide, and
(2) treating the prepared solution with a protein-deamidating enzyme.

A typical aspect of the method for producing a food or a medicine includes the following steps (1) and (2). A deactivation step of the protein-deamidating enzyme may be added after the step (2). Specific conditions of the following steps (1) and (2) are as described in the section "1.1. Protein Deamidation Method" above.

(1) Preparing a solution containing a food material or a pharmaceutical material containing a protein, the solution containing a salt and/or a polysaccharide, and
(2) treating the prepared solution with a protein-deamidating enzyme.

Examples of the solution prepared in the step (1) include a solution prepared by adding a salt and/or a polysaccharide to vegetable milk prepared using a vegetable protein.

The deamidated protein produced by the method of the present invention has a commercial value by itself. On the other hand, it is also useful as an ingredient or a material for various foods and beverages. Therefore, the present application also provides a food or beverage containing the deamidated protein obtained by the production method of the present invention. The food and beverage here are not particularly limited. Examples of the food and beverage include processed marine products (tube-shaped fish paste cake, fish paste cake, fish minced and steamed, shredded and dried squid, dried fish, salted fish guts, a fish sausage, foods boiled in soy sauce, canned food, etc.), processed meat products (ham, bacon, sausage, jerky, corned beef, restructured meat, etc.), processed vegetable products (canned or bottled vegetable, processed tomato, processed mushroom, pickled vegetables, dried vegetables, vegetable boiled down in soy sauce, etc.), noodles and breads (various noodles, bread, a sweet roll, etc.), processed grains (cereal, oatmeal, muesli, processed rice, wheat-gluten bread, barley tea, etc.), dairy products (milk, processed milk, milk beverage, concentrated milk, powdered milk, condensed milk, fermented milk, lactic acid bacteria beverage, yogurt, butter, cheese, ice cream, etc.), processed fruit products (canned or bottled fruits, jam, marmalade, dried fruits, etc.), confectionery and desserts (biscuits, baked pastries, rice confectioneries, fried snacks, Japanese-style uncooked confectioneries, unbaked cakes, semi-perishable confectioneries, Japanese dry confectioneries, candy, chocolate, chewing gum, snack food, jelly, frozen desert, etc.), beverages and the like (soft drink, carbonated drink, fruit juice, coffee-based drink, vegetable juice drink, tea-based drink, nonalcoholic drink, alcoholic drink, etc.), seasonings (soy-based sauce, broth, dressing, sauce, etc.), soups, tofu, noodles, breads, meat substitutes and dairy substitutes made from plant material (cheese substitutes and fermented milk product substitutes), roux (curry roux, stew roux, etc.), nutrition-supplement foods and drinks (protein powder, protein beverage, supplement, nutritional drink, etc.), pet food, and nutritional supplements for pets. Furthermore, it is used in a wide range of industries including cosmetics, medical supplies, microcapsule materials, and carriers for immobilized enzymes and the like as novel protein-derived materials.

3. Agent for Modifying Protein

The present invention further provides an agent for modifying protein that can be used for deamidation of a protein. The agent for modifying protein of the present invention is typically used in the protein deamidation method, the method for producing a deamidated protein, or the method for producing a food or a medicine of the present invention. The agent for modifying protein of the present invention contains a salt and/or a polysaccharide as active ingredients in addition to the protein-deamidating enzyme. Since the details of the protein-deamidating enzyme, the salt, and the polysaccharide are as described above, the description thereof will be omitted. The content of the protein-deamidating enzyme falls within, but is not limited to, 1 to 2,000 U, preferably 10 to 1,500 U, more preferably 50 to 1,000 U, per 1 g of the preparation. The content of the salt is 0.1 to 10 g, preferably 0.2 to 9 g, more preferably 0.5 to 8 g, per 1 U of the protein-deamidating enzyme. The content of the polysaccharide is 0.01 to 10 mg, preferably 0.05 to 5 mg, more preferably 0.1 to 1 mg, per 1 U of the protein-deamidating enzyme. The final form of the agent for modifying protein may be a liquid form or a solid form (including a powder form). The agent for modifying protein may contain an excipient, a buffer, a suspending agent, a stabilizer, a preservative, an antiseptic, a physiological saline, and the like in addition to the active ingredients.

EXAMPLES

Hereinafter, the present invention will be described in more detail with reference to examples, but the present invention is not limited thereto.

<Example 1>

In order to examine whether the deamidation reaction of protein glutaminase is promoted by the presence of the salt, an experiment using pea protein (product name: NOW (registered trademark), manufactured by Sports Organic Pea Protein) as a substrate was used. In purified water, 10 wt% (final concentration) of pea protein, protein glutaminase (product name: Protein Glutaminase "Amano" 500, manufactured by Amano Enzyme Inc.), and 0.5% (W/V%) (final concentration) of sodium citrate were mixed, the pH was adjusted to 7.5 using hydrochloric acid or sodium hydroxide, and then the mixture was incubated at 52.5°C for 1.5 hours to cause a reaction. After completion of the reaction, ammonia liberated by protein glutaminase was quantified as follows. The reaction solution was treated at 85°C for 10 minutes to stop the reaction, 1 mL of the reaction solution was centrifuged (13,000 rpm, 10 min), the obtained supernatant was diluted 100 times with ultrapure water, and ammonia was quantified using Ammonia Assay Kit (Sigma-Aldrich). The amount of the enzyme was set to 7.5 U of protein glutaminase (final concentration) per 1 g of the substrate protein. The deamidation rate was calculated by dividing the amount of ammonia liberated by protein glutaminase per 1 g of pea protein by the amount of ammonia liberated by acidolysis (reaction in 6-N hydrochloric acid (final concentration) at 110°C for 24 hours) of 1 g of pea protein. For comparison, the deamidation rate was measured by performing an enzyme reaction under the same conditions as described above except that sodium citrate was not added.

The results are shown in Table 1. By causing a reaction of protein glutaminase in the presence of sodium citrate, the deamidation rate of pea protein was significantly improved as compared with that in the absence of the salt.

[Table 1]

| Promoter | Deamidation rate (%) | Ratio |
| --- | --- | --- |
| None | 15.4% | 1 |
| 0.5% Sodium citrate | 19.2% | 1.25 |

<Example 2>

It was examined whether the deamidation reaction of protein glutaminase is promoted by the presence of the inorganic salt. The reaction and evaluation were performed in the same manner as in Example 1 except that 1.0% (W/V%) (final concentration) of dipotassium hydrogen phosphate (DKP) was used instead of 0.5% (W/V%) (final concentration) of sodium citrate. For comparison, the deamidation rate was measured by performing an enzyme reaction under the same conditions as described above except that dipotassium hydrogen phosphate was not added.

The results are shown in Table 2. By causing a reaction of protein glutaminase in the presence of DKP, the deamidation rate of pea protein was greatly improved as compared with that in the absence of the inorganic salt.

[Table 2]

| Promoter | Deamidation rate (%) | Ratio |
|---|---|---|
| None | 15.4% | 1 |
| 1% Dipotassium hydrogen phosphate | 20.8% | 1.35 |

<Example 3>

Regarding the effect of promoting the deamidation reaction by the inorganic salt, the influence of the treatment pH and the inorganic salt concentration was examined. The reaction and evaluation were carried out in the same manner as in Example 2 except that pea protein was changed (in the present example, product name: PURIS Pea Protein 870, manufactured by PURIS Foods was used) and that the treatment pH and the concentration of dipotassium hydrogen phosphate (DKP) were changed. The pH was adjusted using hydrochloric acid or sodium hydroxide.

The results are shown in Table 3. In all pH conditions (pH 6.5 to 8.5), regardless of the DKP concentration, the deamidation rate of pea protein by protein glutaminase was improved in the presence of DKP as compared with the absence of DKP.

[Table 3]

| Promoter | Treatment pH | Deamidation rate (%) | Ratio |
|---|---|---|---|
| None | 6.5 | 28.7% | 1 |
| 0.5% Dipotassium hydrogen phosphate | 6.5 | 31.3% | 1.09 |
| 1% Dipotassium hydrogen phosphate | 6.5 | 32.2% | 1.12 |
| 2% Dipotassium hydrogen phosphate | 6.5 | 32.2% | 1.12 |
| None | 7.5 | 27.5% | 1 |
| 0.5% Dipotassium hydrogen phosphate | 7.5 | 29.6% | 1.07 |
| 1% Dipotassium hydrogen phosphate | 7.5 | 30.6% | 1.11 |
| 2% Dipotassium hydrogen phosphate | 7.5 | 33.1% | 1.20 |
| None | 8.5 | 24.0% | 1 |
| 0.5% Dipotassium hydrogen phosphate | 8.5 | 25.1% | 1.05 |
| 1 % Dipotassium hydrogen phosphate | 8.5 | 25.6% | 1.07 |
| 2% Dipotassium hydrogen phosphate | 8.5 | 25.5% | 1.06 |

<Example 4>

It was examined whether the deamidation reaction of protein glutaminase is promoted by the presence of the polysaccharide. The reaction and evaluation were carried out in the same manner as in Example 1 except that pea protein was changed (in the present example, product name: PURIS Pea Protein 870, manufactured by PURIS Foods was used) and that 0.02% (W/V%) (final concentration) of gellan gum was used instead of 0.5% (W/V%) (final concentration) of sodium citrate. For comparison, the deamidation rate was measured by performing an enzyme reaction under the same conditions as described above except that gellan gum was not added.

The results are shown in Table 4. By causing a reaction of protein glutaminase in the presence of gellan gum, the deamidation rate of pea protein was greatly improved as compared with that in the absence of gellan gum.

[Table 4]

| Promoter | Deamidation rate (%) | Ratio |
|---|---|---|
| None | 26.9% | 1 |
| 0.02% Gellan gum | 29.9% | 1.11 |

<Example 5>

It was examined whether the deamidation reaction of protein glutaminase is promoted by the presence of both the polysaccharide and the inorganic salt. The reaction and evaluation were performed in the same manner as in Example 4 except that 1.0% (W/V%) (final concentration) of dipotassium hydrogen phosphate (DKP) was used in addition to 0.02% (W/V%) (final concentration) of gellan gum. For comparison, the deamidation rate was measured by performing an enzyme reaction under the same conditions as described above except that neither gellan gum nor DKP was added.
The results are shown in Table 5. By causing a reaction of protein glutaminase in the presence of gellan gum and DKP, the deamidation rate of pea protein was greatly improved as compared with that in the absence of the polysaccharide and DKP.

[Table 5]

| Promoter | Deamidation rate (%) | Ratio |
|---|---|---|
| None | 25.2% | 1 |
| 1 % Dipotassium hydrogen phosphate + 0.02% gellan gum | 32.1% | 1.27 |

<Example 6>

It was examined whether the deamidation reaction of protein glutaminase is promoted by the presence of both the polysaccharide and polyphosphoric acid. The reaction and evaluation were performed in the same manner as in Example 5 except that 0.5% (W/V%) (final concentration) of sodium polyphosphate (sodium hexametaphosphate) (SPP) was used instead of 1.0% (W/V%) (final concentration) of dipotassium hydrogen phosphate (DKP). For comparison, the deamidation rate was measured by performing an enzyme reaction under the same conditions as described above except that neither gellan gum nor SPP was added.
The results are shown in Table 6. By causing a reaction of protein glutaminase in the presence of gellan gum and SPP, the deamidation rate of pea protein was greatly improved as compared with that in the absence of the polysaccharide and SPP.

[Table 6]

| Promoter | Deamidation rate (%) | Ratio |
|---|---|---|
| None | 27.0% | 1 |
| 0.5% Sodium polyphosphate + 0.02% gellan gum | 31.4% | 1.16 |

<Example 7>

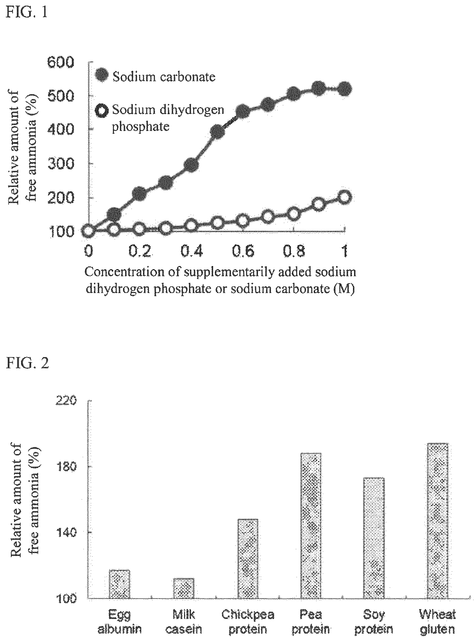

A reaction between Benzyloxycarbonyl-L-glutaminylglycine (Z-Gln-Gly) and protein glutaminase was carried out using a solvent prepared by supplementarily adding sodium dihydrogen phosphate or sodium carbonate to a solution containing disodium hydrogen phosphate, and the influence of the supplementation of sodium dihydrogen phosphate or sodium carbonate on the deamidation reaction efficiency was evaluated. Specifically, Z-Gln-Gly (final concentration: 60 mM) was added to McIlvaine buffer (pH 7.0, containing 16.5 mM of disodium hydrogen phosphate and 1.8 mM of citric acid), and sodium dihydrogen phosphate or sodium carbonate (final concentration of the added portion: 0 to 1.0 M) was further added thereto to prepare a solution having a pH adjusted to 7.0. To this solution, 0.1 ml of an enzyme liquid containing 0.13 U/ml of protein glutaminase (product name: Protein Glutaminase "Amano" 500, manufactured by Amano Enzyme Inc.) was added, and a reaction was caused at 37°C for 20 minutes. Thereafter, 1.0 ml of a 0.4-M trichloroacetic acid solution was added to stop the reaction. Next, the amount of ammonia released by protein glutaminase was measured using an ammonia measurement kit (product name: Ammonia Test Wako, manufactured by FUJIFILM Wako Pure Chemical Corporation). The relative value of the amount of ammonia liberated when sodium dihydrogen phosphate or sodium carbonate was supplementarily added was determined with the amount of ammonia liberated under the condition that sodium dihydrogen phosphate or sodium carbonate was not supplementarily added (that is, when 16.5 mM of disodium hydrogen phosphate alone is contained as the salt) being 100%.
The results are shown in Fig. 1. As a result, it has been found that when sodium dihydrogen phosphate or sodium carbonate is supplementarily added to the solution containing disodium hydrogen phosphate, the deamination reaction efficiency is improved depending on the concentration of sodium dihydrogen phosphate or sodium carbonate. In particular, when sodium carbonate is supplementarily added to a solution containing sodium hydrogen phosphate, the deamination reaction efficiency is remarkably improved, and thus it has been revealed that even when sodium carbonate is used alone as the salt, the deamination reaction efficiency can be improved.

<Example 8>

The deamidation reaction efficiency obtained by performing a reaction between various substrate proteins and protein glutaminase using a solvent obtained by supplementarily adding sodium carbonate to a solution containing disodium hydrogen phosphate was evaluated. Specifically, egg albumin, milk casein, chick bean protein, pea protein, soybean protein, or wheat gluten (final concentration: 1.0 W/V%) was added to McIlvaine buffer (pH 7.0, containing 16.5 mM of disodium hydrogen phosphate and 1.8 mM of citric acid), and sodium carbonate (final concentration: 0.5 M) was further supplementarily added thereto to prepare a solution having a pH adjusted to 7.0. To this solution, 0.1 ml of an enzyme liquid containing 0.13 U/ml of protein glutaminase (product name: Protein Glutaminase "Amano" 500, manufactured by Amano Enzyme Inc.) was added, and a reaction was caused at 37°C for 20 minutes. Thereafter, 1.0 ml of a 0.4-M trichloroacetic acid solution was added to stop the reaction. Next, the amount of ammonia released by protein glutaminase was measured using an ammonia measurement kit (product name: Ammonia Test Wako, manufactured by FUJIFILM Wako Pure Chemical Corporation). A measurement was performed in the same manner under the condition that sodium carbonate was not supplementarily added (that is, when 16.5 mM of disodium hydrogen phosphate alone is contained as the salt), and the relative value of the amount of ammonia liberated when sodium carbonate was supplementarily added was determined with the amount of ammonia liberated under the condition being 100.

The results are shown in Fig. 2. As a result, it was found that the deamination of the substrate protein by protein glutaminase is promoted when 0.5 M of sodium carbonate is supplementarily added to the solution containing disodium hydrogen phosphate. In addition, in the reaction system to which the salt is added, the deamination reaction by protein glutaminase is promoted for both of the vegetable protein and the animal protein, and particularly, it has been revealed that the effect of promoting the deamination reaction is remarkably high for the vegetable protein.

<Example 9>

The thermal stability of protein glutaminase was evaluated using a solvent obtained by supplementarily adding sodium carbonate to a solution containing disodium hydrogen phosphate. Specifically, protein glutaminase (product name: Protein Glutaminase "Amano" 500, manufactured by Amano Enzyme Inc.) was added to a solution of McIlvaine buffer (pH 7.0, containing 41.2 mM of disodium hydrogen phosphate and 4.4 mM of citric acid), supplemented with sodium carbonate (final concentration: 1.0 M) and adjusted to pH 7.0, at a concentration of 0.13 U/ml, and incubated at 50°C for 120 min or 60°C for 60 min, and the activity of protein glutaminase was measured over time. The activity of protein glutaminase was performed by the method described above. The residual activity value of protein glutaminase was determined with the activity value of protein glutaminase before incubation being 100%. For comparison, the test was performed under the same conditions as described above except that 1.0 M of sodium carbonate was not added, and the activity of protein glutaminase was measured over time.

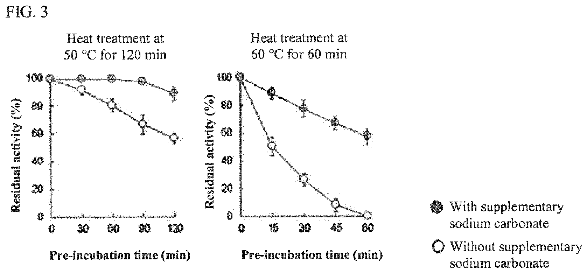

The results are shown in Fig. 3. As a result, it was confirmed that the thermal stability of protein glutaminase was improved in the presence of sodium carbonate.

INDUSTRIAL APPLICABILITY

According to the protein deamidation method of the present invention, the deamidation reaction is promoted, and the reaction efficiency can be expected to be improved. The deamidated protein obtained by the present invention has a commercial value by itself and can also be used as an ingredient or material of various foods, beverages, or medicines. That is, the present invention is expected to be used and applied particularly in the food and beverage field and the pharmaceutical field.

The present invention is not limited to the description of the embodiment and examples of the invention. Various modifications that can be easily conceived by those skilled in the art without departing from the scope of the claims are also included in the present invention. The entire contents of the articles, patent laid-open publications, patent publications, and the like specified in the present specification are incorporated herein by reference.

**Claims**

1.    A protein deamidation method comprising allowing a protein-deamidating enzyme to act on a protein in a presence of a salt and/or a polysaccharide.

2. The protein deamidation method according to claim 1, wherein the salt is at least one selected from the group consisting of carbonates, phosphates, hydrogen phosphates, polyphosphates, and citrates.

3. The protein deamidation method according to claim 1 or 2, wherein the polysaccharide is gellan gum.

4. The protein deamidation method according to any one of claims 1 to 3, wherein the protein-deamidating enzyme is protein glutaminase.

5. The protein deamidation method according to any one of claims 1 to 4, wherein the protein is a vegetable protein and/or an animal protein.

6. An agent for modifying protein comprising a salt and/or a polysaccharide and a protein-deamidating enzyme.

7. The agent for modifying protein according to claim 6, wherein the salt is a phosphate, a polyphosphate, or a citrate.

8. The agent for modifying protein according to claim 6 or 7, wherein the polysaccharide is gellan gum.

9. The agent for modifying protein according to any one of claims 6 to 8, wherein the protein-deamidating enzyme is protein glutaminase.

10. A method for producing a deamidated protein, comprising the steps of:

    (1) preparing a solution containing a protein and containing a salt and/or a polysaccharide; and
    (2) treating the prepared solution with a protein-deamidating enzyme.

11. A method for producing a food or a medicine, comprising the steps of

    (1) preparing a solution containing a food material or a pharmaceutical material and containing a salt and/or a polysaccharide; wherein the food material or pharmaceutical material contains a protein and
    (2) treating the prepared solution with a protein-deamidating enzyme.

12. The method according to claim 10 or 11, wherein the salt is at least one selected from the group consisting of carbonates, phosphates, hydrogen phosphates, polyphosphates, and citrates.

13. The method according to any one of claims 10 to 12, wherein the polysaccharide is gellan gum.

14. The method according to any one of claims 10 to 13, wherein the protein-deamidating enzyme is protein glutaminase.

15. The method according to any one of claims 10 to 14, wherein the protein is a vegetable protein and/or an animal protein.

FIG. 1

FIG. 2

FIG. 3

Heat treatment at 50 °C for 120 min

Heat treatment at 60 °C for 60 min

Residual activity (%)

Pre-incubation time (min)

Residual activity (%)

Pre-incubation time (min)

With supplementary sodium carbonate

Without supplementary sodium carbonate

<div align="center">

### INTERNATIONAL SEARCH REPORT

</div>

| International application No. |
|---|
| PCT/JP2021/014322 |

**A. CLASSIFICATION OF SUBJECT MATTER**
Int.Cl. C12P21/00(2006.01)i, A23L5/00(2016.01)i, A61K38/50(2006.01)i
FI: C12P21/00Z, A23L5/00J, A23L5/00M, A61K38/50

According to International Patent Classification (IPC) or to both national classification and IPC

**B. FIELDS SEARCHED**

Minimum documentation searched (classification system followed by classification symbols)
Int.Cl. C12P21/00, A23L5/00, A61K38/50

Documentation searched other than minimum documentation to the extent that such documents are included in the fields searched
Published examined utility model applications of Japan       1922-1996
Published unexamined utility model applications of Japan     1971-2021
Registered utility model specifications of Japan             1996-2021
Published registered utility model applications of Japan     1994-2021

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
|---|---|---|
| X | WO 2010/035825 A1 (AJINOMOTO CO., INC.) 01 April 2010 (2010-04-01), claims 1-5, paragraphs [0023], [0024], [0040]-[0043], [0047]-[0049], [0064] | 1-15 |
| X | JP 2019-208371 A (TAIKI-BUSSAN CORP.) 12 December 2019 (2019-12-12), claims 3, 7, 11, paragraph [0062] | 1-3, 5-8, 10-13, 15 |
| A | 森口 充瞭, 旨味を増強する酵素・グルタミナーゼ, 日本醤油研究所雑誌, vol. 28, no. 1, pp. 1-12, particularly, p. 2, table 2, (MORIGUCHI, Mitsuaki, Umami-enhancing enzyme·Glutaminase, Journal of Japan Soy Sauce Research Institute) | 1-15 |

☐ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 May 2021 | 18 May 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

| INTERNATIONAL SEARCH REPORT | International application No. |
| Information on patent family members | PCT/JP2021/014322 |

WO 2010/035825 A1  01 April 2010       US 2011/0236529 A1
                                       claims 1-5, paragraphs [0044],
                                       [0045], [0068], [0069], [0073],
                                       [0074], [0083]-[0086]
                                       EP 2332421 A1
                                       CN 102164496 A

JP 2019-208371 A   12 December 2019    (Family: none)

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000050887 A
- JP 2001218590 A
- WO 2006075772 A
- WO 2015133590 A
- WO 2008000714 A